Europäisches Patentamt

(19) European Patent Office

Office européen des brevets

(11) Publication number: **0 053 903**
**B1**

(12) **EUROPEAN PATENT SPECIFICATION**

(45) Date of publication of patent specification: **22.04.87**

(51) Int. Cl.⁴: **A 61 C 8/00**, A 61 K 6/08, A 61 L 17/00

(21) Application number: **81305646.2**

(22) Date of filing: **30.11.81**

(54) **Oral prosthesis and method for producing same.**

(30) Priority: **08.12.80 US 214572**

(43) Date of publication of application:
**16.06.82 Bulletin 82/24**

(45) Publication of the grant of the patent:
**22.04.87 Bulletin 87/17**

(84) Designated Contracting States:
**AT BE CH DE FR GB IT LI LU NL SE**

(56) References cited:
**DE-A-2 255 916**
**FR-A-2 105 998**
**US-A-3 628 248**
**US-A-4 199 864**

(73) Proprietor: **MEDICAL BIOLOGICAL SCIENCES, INC.**
**200 Central Park South**
**New York New York 10019 (US)**

(72) Inventor: **Bruins, Paul Fastenau**
**390 Rugby Road**
**Brooklyn New York 11363 (US)**
Inventor: **Ashman, Arthur**
**200 Central Park South**
**New York New York 10019 (US)**

(74) Representative: **McCallum, William Potter Cruikshank & Fairweather 19 Royal Exchange Square**
**Glasgow G1 3AE Scotland (GB)**

Courier Press, Leamington Spa, England.

### Description

This invention relates to an oral prosthesis and the process for producing it. More particularly it relates to porous implants which allow for the growth of bone and gum tissue into the implant to assure that it is firmly attached to the body structures and becomes an integral part or fixation thereof. It further relates to molding compounds for such implants. It also relates to a process for rapidly molding such implants to replace the root of an extracted tooth, an entire tooth or a section of bone with an identically shaped replica within a short period of time after extraction or during a single office visit.

Oral prosthetic devices, in the form of dental implants are one class of biologically compatible components used in the body to replace injured or diseased body structures. Such components are biologically compatible in the sense that implantation of such devices has no adverse effect upon the living tissue, and the environment of the body does not adversely affect the device. It may be necessary to replace entire bony structures, fill bone defects, or replace extracted teeth.

The replacement of a bone section or a tooth offers the particularly challenging problem of finding a biologically compatible material which will allow tissue growth to penetrate into the material.

U.S. Patent No. 4,186,448 to Brekke discloses a device and method for treating a newly created bone void or soft tissue deficiency. A body made of a biodegradable material, such as polylactic acid, including enclosed interconnected randomly-positioned, randomly-shaped and randomly-sized voids extending throughout the mass of the body attracts blood by capillary action, leading to healing of the bone void as the body member biodegrades and is absorbed by the tissue. A wetting agent such as sodium oleate or Ultrawet 60L (T.M.) sold by the Fisher Scientific Company of Chicago, Illinois assists the capillary action (see Column 1, lines 55—62). This type of implant, while promoting healing and possibly preventing the bone structure around an extracted tooth from receding, does not provide a structure to which replacement crowns for extracted teeth can be attached.

"Fabrication and Characterization of Porous-rooted Polymethylmethacrylate (PMMA) Dental Implants," by Klawitter et al. published in the *Journal of Dental Research*, April 1977, p. 385 discloses polymethylmethacrylate dental implants having a porous coating on the root portions. The coating is developed by adding methylmethacrylate monomer to polymethylmethacrylate beads to soften the surface of the beads, and then compression molding the mixture at high pressure. Pore sizes of 20—40 micron diameter are indicated as being restrictive of bone growth but allowing for fibrous tissue ingrowth, while those of approximately 200 micron diameter are disclosed as appearing to be optimum for bone tissue ingrowth.

U.S. Patent No. 4,199,864 to Ashman discloses a method for fabricating polymeric plastic implants for endosteal or periosteal applications having a porous surface with pores of a predetermined size, pore depth, and degree of porosity. Leachable substances, such as sodium chloride crystals of controlled particle size are added to a powdered polymer-liquid monomer mixture in relative amounts corresponding to the desired degree of porosity. These crystals, combined with mold release agents, are used to coat mold cavity surfaces to achieve proper near-surface porosity. After heat polymerization without the use of an initiator, and abrasive removal of resulting surface skin, the salt is removed by leaching to provide the desired porosity. Bone ingrowth is promoted by pore sizes in the 200—400 micron range. Pore sizes of 50—150 microns result in soft tissue ingrowth. The use of two different size pores in a single implant is disclosed at column 3, lines 29—36.

According to the present invention there is provided a molded, non-biodegradable porous implantable oral prosthesis having an area of relatively coarse porosity where the prosthesis is intended to interface with bone tissue and relatively fine porosity where the prosthesis is intended to interface with soft tissue characterised in that the prosthesis comprises sintered polymeric particles coated with a hydrophilic material.

According to a further aspect of the present invention there is provided a process for producing a porous implantable oral prosthesis comprising the steps of:

a) filling a portion of a mold cavity of the desired shape with relatively coarse polymeric particles coated with a hydrophilic monomer where said prosthesis is intended to interface with bone tissue;

b) filling the remainder of said mold cavity with relatively fine polymeric particles coated with a hydrophilic monomer where said prosthesis is intended to interface with soft tissue;

c) sintering the polymeric particles and polymerizing the hydrophilic monomer in the mold by the application of heat to form the prosthesis;

d) allowing the mold to cool;

e) removing the sintered prosthesis from the mold; and

f) placing the prosthesis in a hot liquid to remove toxic residues.

The present invention provides an oral prosthesis comprised of a porous biologically compatible material. Unlike the Brekke patent cited above the implants of the present invention are made of a nonbiodegradable material, having specific pore sizes per unit area, which are not randomly spaced throughout and requires not wetting agent. A molding compound comprised of particles of the material, which is preferably a biologically compatible polymeric plastic, such as polymethylmethacrylate (PMMA), is combined with a monomeric hydrophilic material which physically coats the particles, and is polymerized while sintering in a dielectric oven. The PMMA may be of a modified form including a plasticizer or a comonomer. The polymerized hydrophilic material facilitates the infusion of body fluids into the implant, which is coarsely porous where it must interface with bone tissue, and finely porous where it must interface with gum tissue.

The hydrophilic material is advantageously a lightly inhibited monomer, such as hydroxyethylmethacrylate (HEMA) which physically coats. but does not chemically interact, with the polymeric material. Heating provides sufficient free radical activity to overcome the effect of the inhibitor, resulting in polymerization of the coating and the bridges between the particles during sintering, thus assuring adequate strength. Post sintering immersion for a brief interval in boiling water sterilizes the implant and removes residual monomer and inhibitor. Thus, the final step in the process of producing the implant renders it sterile.

The hydrophilic coatings swell but do not generally dissolve in body fluids. In order to assure that dissolution is prevented, a small quantity of cross-linking agent may be added to the polymer-monomer mixture, thus increasing the effective molecular weight of the polymerized hydrophilic material.

When a tooth is extracted it is desirable to fill the void created to prevent recession of the gum and bone. It is necessary, if a replacement crown is to be provided, to have a root implant to which the crown can be fastened, and which will adequately support the crown. Further, for best results and strong attachment to bone and gum tissues, the implant should be placed in the extraction cavity almost immediately after extraction. It should be of the same size and shape as the root of the extracted tooth, or possibly just slightly larger in size to fit snugly into the cavity.

The present invention provides a method of fabricating a replica of the root of the extracted tooth within a very short time after extraction, allowing the root implant to be inserted into the extraction cavity during the same office visit as the extraction. Using the cleaned, extracted tooth, a mold is produced which is a replica of the root section of the tooth. It is filled by packing with molding compounds comprised of different sizes of polymer particles to provide the two required sizes of pores. Sintering is accomplished in minutes by heating in a dielectric oven. After cooling, removal from the mold and immersion in a hot liquid to remove impurities, the implant is cut down in size so it may be completely enclosed and covered by the patient's gum tissue (except for possibly a part protruding through the gum (where it remains for several weeks or months to allow the ingrowth of bone and gum tissue before a crown is mounted.

To produce a snugly fitting root implant the cleaned, extracted tooth is immersed in molten wax and withdrawn, allowing a thin conformal coating of wax to solidify on the root. A replica produced by using this wax coated tooth root will be of the proper shape but slightly larger than the original root. This will not affect the ability to insert the replica as there is some latitude in the size of an implant which the bone and tissue can accept.

A prefabricated denture tooth such as that made by denture manufacturers may be attached to an implanted root made by the present invention or the process of the present invention can be used to produce an entire tooth when necessary or desirable. To do so it is necessary to prepare a mold of the crown, or upper portion of the tooth, as well as the root portion. The extracted tooth which is to be replicated remains in a mold of the type described above. This mold, with the tooth in place, is used to produce a mating mold having a cavity in the shape of the crown. After the molds have been disassembled and the patient's tooth removed, a porous root is then produced in the root mold by the process described above. The second mold cavity is filled with a material such as a fast setting polymer formulation which polymerizes to form an artificial crown. As soon as this crown mold cavity of the mating mold is so filled, the root mold, with the porous sintered root in place in its mold cavity is placed in proper alignment above the crown mold. Penetration of the liquid into the porous root occurs as the liquid sets. Air pressure may be used to prevent the formation of voids during the cure of the polymer formulation. A post cure in a dielectric oven may be utilized to assure completion of polymerization. After cooling the molds are disassembled from one another, and the complete artificial tooth removed. Mold parting line flash is removed from the tooth, which is then immersed in boiling water to remove impurities. The complete tooth can then be secured in the cavity in the mouth by attachment to adjacent teeth by acid-etch and "bonding" techniques, while tissue grows into the root portion.

Where bone and gum tissue have receded due to prior extractions an anchor for partial dentures or a bridge which may be implanted can be fabricated by drilling a conical cavity in the patient's jaw bone, making an impression of this cavity, using the impression to generate a mold, and then using this mold to produce a porous article of the shape of the conical cavity following the procedure outlined above for producing a tooth root. This anchor is provided with a central post projecting above the gum line to which a partial denture, a bridge or a crown may be attached.

Embodiments of the present invention will now be described, by way of example, with reference to the accompanying drawings in which:—

FIG. 1 is an enlarged cross sectional, side elevation of a crown and a tooth root made in accordance with the invention showing the pores in greatly enlarged scale.

FIG. 2 is an enlarged cross sectional side elevation of a porous implant made in accordance with the invention attached to a crown by means of a post.

FIG. 3 is an enlarged cross sectional view of a porous implant including a temporary post made from a non-polar plastic.

FIG. 4 is a side elevation in partial cross sectional of a jaw bone implant made according to the invention.

FIG. 4A is a cross sectional side elevation of a jaw bone implant used when a larger region of bone has deteriorated.

FIG. 5 is a cross section of the jaw bone implant taken along line 5—5 of Fig. 4.

This invention may be used to produce a variety of oral prostheses. Various types are disclosed and illustrated in U.S. Patent 4,199,864 for "Endosseous Plastic Implant Method" to Ashman.

A prosthesis may be produced by molding to predetermined sizes and shapes to fit a particular patient. The cavity of an appropriate mold is packed initially with coarse polymeric particles, which are coated with a hydrophilic material. In the process of the invention it is only necessary to pack the material utilizing strong manual pressure. A quantity of this material sufficient to form a portion of the implant which will interface with bone tissue is utilized leaving a portion of the mold cavity unfilled. This remaining volume is packed in a similar fashion with fine polymeric particles which are coated with a hydrophilic material, forming a portion of the implant which will interface with gum tissue. The packed mold is heated at atmospheric pressure in a dielectric oven where sintering of the PMMA and polymerization of the HEMA in the mold cavity occurs. After the mold has cooled, aided by immersion in cold water, the intered prosthesis is removed from the mold and immersed in a hot liquid for a short period of time.

Occasionally it is desirable to produce an implant which is an exact replica of a patient's whole tooth. If an exact replica can be inserted into the cavity formed with a diseased tooth is extracted, within a short time after extraction, i.e. within the same orifice visit, ingrowth of bone and gum tissue is facilitated and the implant will become an integral part of the tissue structure. An implant of this variety may be produced by the process outlined below if the extracted tooth is cleaned and used to form a mold comprised of a rapidly setting material. Setting of the mold may be accelerated by dielectric heating. The mold is formed in a matter of minuates and may then be packed, as described above, the material sintered, and a replica of an extracted tooth root produced according to the invention within a period of approximately fifteen minutes to one half hour.

A mold for the upper portion of the tooth is prepared after the mold for the root portion has been made, with the patient's extracted tooth as the pattern, in place in the root mold. A parting agent, such as petroleum jelly is used to coat the top surface of the root mold. Then the rapid setting mold material is used to make a replica of the upper crown portion of the patient's tooth. After cure, (requiring two minutes of dielectric heating) the upper and root portions of the mold are separated. After the porous root portion of the tooth is made, as described above, the crown portion of the mold is filled with a non-porous, fast setting methylmethacrylate (MMA) formulation containing PMMA particles, pigment, filler, caralyst and promoter, mixed with monomeric MMA, a conventional formulation commercially available. While this mixture is still liquid in the crown mold, the root portion of the mold, containing the sintered porous root, is placed on top of the crown mold in perfect alignment, so that the sintered root, with or without a center post of PMMA, contacts the liquid mixture in the crown mold. The liquid portion is converted to solid PMMA, with or without additional dielectric heating. When the root and crown molds are separated, a replica of the patient's tooth, having a porous root and a non-porous crown may be removed from the mold, ready to be inserted into the patient's cavity. Total elapsed time is less than one hour.

Any prosthetic device produced according to this invention may be reinforced by inserting a solid polymer core, such as PMMA into the material packed within the mold cavity.

As indicated above, the dental implants of this invention are produced from materials which can be implanted in living body tissue without adverse effect on the body or the implant. The materials must be easily molded to a particular size and configuration, appropriate for the individual need of a dental patient. The molded implant must have adequate strength to withstand the loads of normal use and occasional abuse. The materials should also be low in cost and readily available. Recent developments in the science of biologically compatible materials have resulted in larger numbers of materials which meet these requirements and more materials are being accepted for use within the body.

A class of materials which has been accepted for use in body tissue for many years is the acrylics. Of these, the material with some of the best mechanical properties is polymethylmethacrylate (PMMA). While it will be understood that the present invention may be practised by using a variety of base materials, the discussion will be restricted to the use of PMMA. The sintering of PMMA particles to produce a porous implant is not broadly new. Such a structure, however, is often not readily penetrated by body fluids due to the hydrophobic nature of untreated PMMA particles.

A highly effective method of assuring adequate penetration, and therefore sufficient ingrowth of body tissue, an important feature of this invention, is the coating of the PMMA particles with a hydrophilic material. While many hydrophilic materials could conceivably be used, it has been found that polyhydroxyethylmethacrylate (PHEMA) is an excellent coating. Because of its hydrophilic nature, it will swell on exposure to body fluid but will not generally dissolve in these fluids.

The preferred method for obtaining a PHEMA coating on the PMMA particles is to use HEMA monomer to wet the PMMA particles just prior to packing the mold. A mixture of approximately ten percent by weight HEMA and ninety per cent PMMA is the optimum mix ratio. This ratio may be varied however from approximately five percent to twenty percent, a slight excess of HEMA being preferred if the optimum ratio is not used. The combining of these ingredients to form a molding compound is preferably and most conveniently carried on at room temperature but could be carried on at any temperature in the range of 5°C to 35°C.

The HEMA monomer, in liquid form at room temperature, should be lightly inhibited from polymerizing by trace amounts of an inhibitor such as the methyl ether of hydroquinone (MEHQ). This should be present in a concentration in the range of 150 to 300 parts per million and, preferably, 200 parts per million. It has been found unnecessary to add any material to neutralize the inhibitor to allow polymerization of the HEMA. Raising the temperature during the sintering process provides sufficient free radicals to allow polymerization to occur.

The polymerized HEMA coating will physically bond to the PMMA particles but will not chemically react with the PMMA. This bond is sufficiently strong to aid in preventing physical breakdown of the sintered structure. To further assure against dissolution of the coating, a cross linking agent comprising a methacrylic diester of ethyleneglycol may be added to the inhibited HEMA-PMMA mixture to increase the effective molecular weight of the polymerized HEMA coating. This cross linking agent may comprise one tenth percent to five percent by weight of the HEMA monomer. It is preferably triethyleneglycol dimethacrylate or alternatively tetraethyleneglycol dimethacrylate. Diethyleneglycol dimethacrylate and monoethyleneglycol dimethacrylate may also be used. Combinations of these four diesters with a combined weight from one tenth percent to five percent of the HEMA may be used. HEMA monomer usually contains a small amount (1% or less) of monoethyleneglycol dimethacrylate initially, unless specifically purified, because this· diester forms spontaneously in HEMA by the process of transesterification.

After two or three minutes of mixing the inhibited HEMA-PMMA mixture, with or without a cross linking agent, the PMMA particles become tacky and the HEMA physically penetrates into the PMMA surface, resulting in an excellent welding action during subsequent sintering, which produces a stronger implant than one resulting from the sintering of only PMMA particles. Where the HEMA coated particle surfaces meet, the polymerization and cross linking of the HEMA, which is chemical in nature, contributes to this result, producing a porous implant of exceptional strength.

The size of the pores may be controlled by the size of the PMMA particles used. It is generally convenient to use commercially available beads of 40 to 60 U.S. mesh, which correspond to a particle diameter of about 250 to 420 microns, to produce pore sizes of 125 to 210 microns, which are suitable for soft tissue ingrowth. Beads of 20 to 24 U.S. mesh, corresponding to particle diameters of approximately 700 to 840 microns, produce pore sizes of 350 to 415 microns, which are suitable for bone ingrowth. Such PMMA beads are made by suspension polymerization and are spherical in shape. They are made by Esschem Co. of Essington, Pennsylvania and are called Polymer Type 5, which indicates that they are comprised of five percent dibutyl phthalate.

To produce a dental implant having a portion designed to interface with bone or hard tissue including large or coarse pores, and a portion designed to interface with gum or soft tissues having small or fine pores, as defined above, it is necessary to pack a mold of the described dental implant with molding compounds produced with PMMA particles of the above indicated sizes. For example, generally the lower portion of the mold, as in the case of a root implant, will produce that part of the implant which will interface with bone tissue. The mold is thus packed with a molding mixture as described above containing PMMA particles in the diameter range of 700 to 840 microns. A sufficient quantity of material is used to fill the mold to the point that material which is tamped in by using manual pressure fills the portion of the implant which will be associated with bone tissue where ingrowth and adhesion of periodental membrane tissue is desirable. The remaining portion of the mold is then filled with a molding compound as described above, having PMMA particle diameters in the range of 250 to 420 microns suitable for soft tissue ingrowth. This material is also tamped in by hand.

The molds into which the molding compound is packed may be of any type known in the prior art which will withstand the stress of manual tamping without undue deformation and will not be adversely affected by exposure to temperatures necessary to sinter the molding compound. Under certain circumstances it may be necessary to spray the surface with a release agent but this is generally not necessary with the preferred molding materials. The preferred material for the mold, which sets rapidly, especially when dielectrically heated, is Citricon silicon putty mixed with Citricon catalyst both well known and manufactured by the Kerr Dental Products Division of Sybron located in Romulus, Michigan. The procedure for using this material is described below.

The material packed in the mold is sintered by heating in a dielectric oven such as a Model E0-1 manufactured by W.T. Rose and Associates of Troy, N.Y. at a temperature in the range of 150°C to 225°C for three to six minutes, but preferably four minutes. The oven's upper electrode is adjusted to provide one quarter inch clearance between it and the top of the mold. Temperatures in this range create enough free radicals to overcome the effect of the inhibitor in the HEMA monomer, resulting in polymerization of the physically bonded HEMA coatings and HEMA bridges between the PMMA particles where they are in contact with one another. Cross linking also occurs when an appropriate cross linking agent, as specified above, is used.

After sintering the mold is removed from the dielectric oven and cooled by partial immersion in water. The cooling is allowed to progress until the implant gains sufficient strength to allow it to be removed from the mold without damage. The mold may be split to facilitate removal.

The resulting implant will generally contain unreacted HEMA monomer or, possibly, exceedingly small amounts of inhibitor. These materials may be removed by immersion of the implant in a hot liquid which will leach out the residue without damaging the implant or leaving behind components of its own. Immersion of the implant in boiling water for one and one-half to two and one half minutes is usually adequate.

The portion of the volume of the finished implant which constitutes the pore volume is advantageously approximately thirty percent and is preferably uniformly distributed, but it may be between twenty percent and forty percent. Lower percentage porosity will not allow adequate infusion of body fluids necessary for tissue ingrowth, and higher percentages of porosity will decrease the strength of the implant.

It is desirable to implant a replacement tooth root which is a replica of the extracted tooth root before the gum and bone tissue can recede away from the extraction site. This promotes rapid healing of the area, a firm bond between the body tissue and the implant, and helps reduce the possibility of a rejection of the implant by the tissue. The present invention can be utilized to produce such a replica, which has coarse porosity where it fits within the jawbone socket interfacing with hard tissue. In such areas the ingrowth or adhesion of bone or periodontal membrane tissue is necessary. In areas which interface with soft gum tissue, finer porosity is advantageously utilized to produce tissue ingrowth.

A method for utilizing the present invention to make a replica of the root of the extracted tooth is set out below. This procedure leads to the production of a replica within approximately twenty minutes of tooth extraction. Thus the implant may be inserted in the patient during the office visit in which the tooth is extracted.

Example 1

Procedure for replacement of a newly extracted tooth by an implantable root.

A tooth root may be rapidly molded by following the steps outlined below:

1) The tooth is extracted and cleaned of material not part of the tooth. (Elapsed Time — 1 minute)

2) Twenty-five grams "Citricon" silicon putty is mized with ten drops "Citricon" catalyst for one minute and pressed into a polyethylene ring, 2.54 cm (one inch) in inner diameter by 2.54 cm (one inch) high on a glass plate. The mixture is packed well to eliminate voids and excess material is trimmed. (Elapsed Time — 1.5 minutes)

3) The extracted tooth is pressed into the silicone putty up to and slightly beyond the point representing the gum line. The resulting mold is placed in a dielectric heating oven. The upper electrode is adjusted to be 0.63 cm (one quarter inch) above the top of the tooth. The mold material is dielectrically heated for two minutes. The tooth is removed from the mold with a rocking motion. (Elapsed Time — 3 minutes)

4) In a small beaker 0.4 gms of 20 to 24 U.S. mesh acrylic (PMMA) beads are thoroughly mixed with 2 drops (.04 gms) of HEMA (hydroxyethylmethacrylate mixed with 1% of triethyleneglycol dimethacrylate). In another small beaker 0.6 gms of 40 to 60 U.S. mesh acrylic beads are thoroughly mixed with 3 drops (.06 grams) of HEMA (also containing the triethyleneglycol dimethacrylate) for one minute. This may be done while the silicone mold is heating and curing, (Elapsed time — 1 minute)

5) The lower part of the silicone mold cavity is filled with the 20 to 24 U.S. mesh mixture, forcing it down, with a spatula, into the root channels to fill about 1/3 to 1/2 of cavity depth. The walls of the cavity above this point are cleared of the 20 to 24 U.S. mesh particles. (Elapsed time — 1 minute)

6) The remainder of the mold cavity is filled with the 40 to 60 U.S. mesh mixture. The material in the mold is pressed down as hard as possible by hand, using a polyethylene film to avoid hand contact. The filled cavity should be level with the top of the mold. Excess is trimmed away. (Elapsed time — 2 minutes)

7) The filled mold is placed in a dielectric oven, with the upper electrode adjusted to be one quarter inch above the top of the mold. Heat is applied for four minutes. The mold is removed and cooled by semi-immersion in water for 2 minutes. (Elapsed time — 6 minutes)

8) The silicone mold is removed from the polyethylene sleeve, which is reuseable. The molded tooth root is gently prised out so as not to damage the long curved root ends, or the mold may be split to remove a complexly shaped root after making sure that the root is cool enough to be strong. (Elapsed time — 2 minutes)

9) The tooth root is placed in boiling water for two minutes to extract any residual monomer and inhibitor. (Elapsed time — 2 minutes)

10) The top of the root is trimmed to the desired height so that when inserted in a patient's cavity it can be completely enclosed and covered by the patient's gum tissue. (Elapsed time — 2 minutes).

Total elapsed time is equal to 21.5 minutes.

It is also possible to make the porous root slightly larger than the removed tooth root for a tighter fit. Dipping the extracted tooth in a molten wax, in order to coat it uniformly, and allowing it to solidify enlarges all dimensions slightly.

After the bone and tissue have become attached (estimated to take 2—6 weeks) the dentist can attach the upper part of the tooth or crown by conventional means after making the necessary incision in the gum to reach the implant or prosthesis. The shape and position of the crown may be duplicated by means of an impression taken prior to the extraction.

In order to facilitate attachment of the crown to the implanted root it is often desirable to provide a temporary, centered rod or post in the porous root. This post may be made of high density polyethylene or polypropylene or other non-polar plastics, of 0.16—0.32 cm (one sixteenth to one eight inch) in diameter, depending upon the size of the root implant. The lower portion of the post may be threaded to facilitate subsequent removal. This post is inserted into the packed porous implant prior to sintering as in step No. 7 of Example 1. Because it is non-polar, it does not become heated in the dielectric field and it does not becomes bonded to the sintered PMMA particles.

Prior or subsequent to insertion of the sintered root implant in the patient's tooth cavity, the height of the post is adjusted by trimming, so that the top of the post is at the same level as or protruding slightly above the top of the gum, and the gum is sutured around the post, so that only the top of the post is visible or slightly protruding.

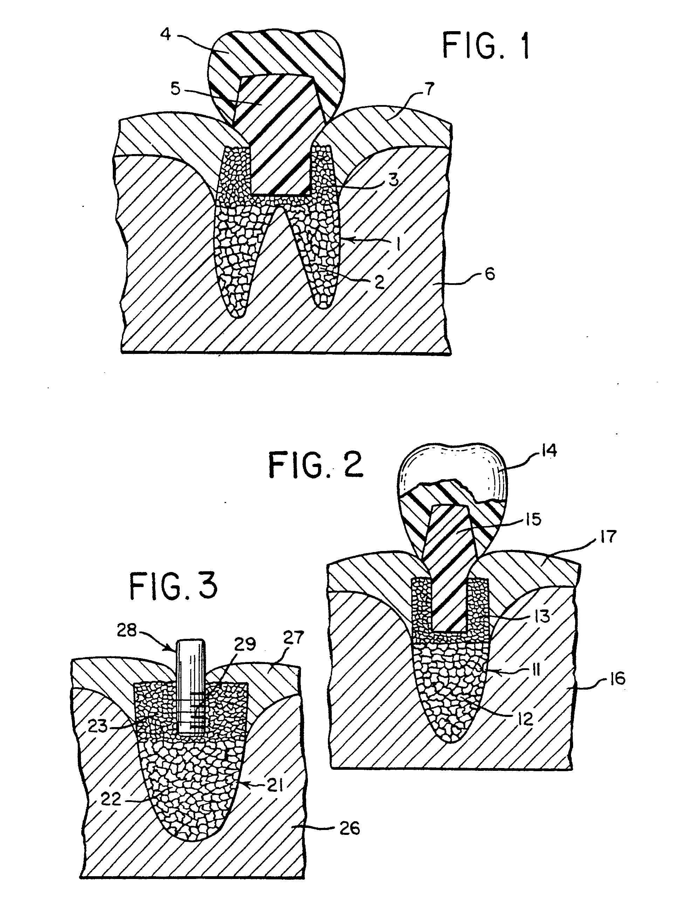

After the bone and tissue have become attached, the dentist can now readily locate the exact position of the implant, and remove the temporary post by unscrewing it, without disturbing the implant. The resulting hole in the implant is now used to insert a PMMA non-porous post or shaped post as shown in Figure 1. The PMMA post need not be threaded. It can be firmly attached to the implant by means of a fast setting acrylic or cyanoacrylic adhesive.

Referring to Fig. 1, there is illustrated an implantable root 1, showing an area of coarse porosity 2 which interfaces with bone 6, and an area of fine porosity 3 which interfaces with gum tissue 7. A post 5 is provided as a means of strengthening root 1 and mounting a crown 4, by the use of a suitable adhesive well known in the art. It is understood that the porosity extends throughout the entire root, and that the entire surface is porous. Post 5 is formed from a solid acrylic (PMMA) and may be inserted in the mold prior to sintering, immediately thereafter, or after the removal of the temporary non-polar post. It may be formed from a rod or a shaped post having a base 0.16—0.32 cm (one sixteenth to one eighth inch) in diameter depending on the size of the patient's tooth.

Fig. 2 illustrates a replacement structure 11 for a smaller tooth, with possibly a less complex root structure. Areas of coarse porosity 12 which interfaces with bone 16 and fine porosity 13 which interface with gum tissue 17 are shown. Crown 14 is attached to root implant 11 by means of post 15 which has a base one sixteenth inch in diameter.

Fig. 3 illustrates an implantable root 21 with an area of coarse porosity 22 which interfaces with bone 26 and fine porosity 23 which interfaces with gum tissue 27. Root 21 is made with a temporary post 28 of a non-polar plastic which is readily removable from the root by means of thread 29 in order to subsequently replace it with a PMMA post.

A method of utilizing the present invention to make a replica of the entire extracted tooth is set out below. This procedure leads to the production of a replica of the entire tooth within one hour or less. Thus, the complete replacement of the patient's extracted tooth can be accomplished during the office visit in which the tooth is extracted.

Example 2

Procedure for replacement of a newly extracted tooth by an implantable tooth.

1) The patient's extracted tooth is inserted in the root mold previously made by the procedure described in Example 1. The top surface of the mold should be flat and at a position corresponding to the gum line of the tooth. Notches are cut into the top surface in order to later align the mold of the crown with the root mold. The root mold is inserted in the original polyethylene ring in which it was previously made.

2) The top surface of the root mold is coated with a thin layer of petroleum jelly or similar lubricant which acts as a parting agent.

3) A second polyethylene ring of the same diameter is placed on top of the root mold ring, the second ring having a height equal to the height of the patient's tooth projecting above the root mold surface, plus an additional 0.65 cm (one quarter of an inch).

4) Sufficient "Citricon" silicon putty and "Citricon" catalyst, are mixed (for example twelve and one half grams of putty and 5 drops of catalyst), for one minute, and pressed into the upper polyethylene ring, filling the ring completely. The ring is packed well to eliminate voids, and excess material is trimmed. The molds are heated dielectrically for two minutes, with the top electrode of the dielectric oven 0.65 cm (one quarter inch) above the top of the molds, to complete the cure of the silicone mold. The molds are then cooled for one minute by immersion in water. The top and bottom molds are separated and the patient's tooth removed.

5) The crown mold is now filled with a fast setting MMA liquid formulation consisting of PMMA powder blended with pignment, filler, catalyst, promoter and monomeric MMA, lightly inhibited, which is usually proportioned to be two parts powder to one part monomer. This mixture of powder and liquid is blended and mixed for thirty seconds and then used to fill the crown mold. The above described formulation is commercially available in various tooth color shades, for example, "Jet Acrylic" manufactured by Lang Dental Mfg. of Chicago, Illinois or "Super-C" manufactured by Amco, Inc. of Philadelphia, Pennsylvania. Such formulations complete the hardening reaction at room temperature (70°F) in about five minutes.

6) As soon as the crown portion of the mold is filled with the above liquid mixture, the root mold, with the porous sintered root inserted in place, and made by the previously described procedure, is placed above the crown mold, using the molded in notches for perfect alignment. The root mold contacts and absorbs some of the liquid mixture in the crown mold. As the porous root contacts the liquid mixture, the excess liquid mixture is squeezed out at the parting line. A clamp is used to hold the two molds together while the liquid mixture is hardening.

Optionally, the porous sintered root may have a center post of a solid polymer core preferably made of PMMA, which is bonded to the porous sintered root, and projects into the liquid mixture. The monomeric MMA in the liquid mixture softens the PMMA post surface and then polymerizes, thus forming a strong bond.

Optionally, as soon as the two molds are joined and clamped, the assembly may be placed in a pressure vessel, and the pressure raised by means of a compressed fluid, such as air, to 60 psi or more while the liquid mixture is converting to a solid. The purpose of this additional pressure is to reduce or completely prevent formation of any voids in the crown portion of the tooth caused by vaporization of monomer due to the exothermic heat of the polymerization reaction.

7) The crown and root portions of the molds are separated, and the completed replica of the patient's tooth removed from the mold. Any "flash" or excess hardened material at the interface is removed, and the crown surfaces may be buffed and polished.

8) The complete finished tooth is placed in boiling water for two minutes to remove residual monomer or inhibitor from the porous root portion. This is done only after the entire tooth is made.

9) The replica of the patient's tooth is inserted into the cavity formed by the extraction in the patient's mouth. It must remain immobile in order to permit bone and tissue attachment. This may be accomplished by several means, for example, wire attachment to adjacent teeth. When a visible front tooth is replaced, a less noticeable form of attachment may be used. For example, a fast setting transparent adhesive such as "Cyanodent" made by the Ellman Dental Laboratories of Hewlett, New York may be used. This material is made with a cyanoacrylic monomer similar to Eastman 910, made by Eastman Kodak, and well known for its use as an adhesive.

Where teeth have been extracted for some time and the bone and gum tissue have receded, a region known as the "edentulous" area forms and there is a need to create an anchor for partial dentures or a bridge. Such an anchor can be provided in the form of an implant which attaches to the bone and tissue and is produced by the following procedure:

Example 3

Procedure for producing an implantable root or entire tooth when the bone and gum have receded due to extraction at some previous time.

1) The gum is cut open at the site for the implant and a conically shaped hole drilled in the bone, using progressively larger shaped drills adapted for use with a dental drill. The finished hole should be about one quarter inch in diameter at the bone surface, and tapered, with a total depth bringing it no closer than two millimeters from a nerve. The position of the nerve is determined by well known x-ray techniques.

2) An impression of the drilled hole is made, using a fast setting, very low shrinkage acrylic formulation, such as "Duralay" made by Reliance Dental Mfg. Co. of Worth, Illinois. This impression is used as a model to make a "Citricon" silicone mold.

3) The porous implantable root is then made as described in Example 1, or a whole tooth is made as in Example 2 using the silicon mold to reproduce the shape of the drilled hole. That portion of the root in contact with bone is made having a pore size of 350 to 415 microns, while the portion in contact with gum tissue is made having a pore size of 125 to 210 microns.

4) The implant is placed in the prepared bone socket, and the gum tissue is sutured around the temporary or permanent post. The implant is then left undisturbed for two to six weeks until bone and tissue attachment has occurred. If the post is the temporary type, it is removed and replaced with a PMMA post preferably of the conical type shown in Fig. 1.

5) The conical projecting portion of the post is then used for attaching the crown portion, or a bridge. The crown is a molded tooth having a socket which conforms to the shape of the conical post and is cemented in place using fast setting acrylic adhesive.

While the invention has been described as using polymethylmethacrylate particles coated with hydroxyethylmethacrylate, it is understood that other biologically suitable materials may become available for use with the present invention.

When an implant produced according to the present invention is placed in a cavity in a patient's jaw it is desirable that it be possible to obtain meaningful data on progress as time goes on by the use of X-ray techniques. The HEMA coated PMMA of the implant will not generally have an X-ray density sufficient to provide meaningful contrast on X-ray film. A radio-opaque material such as barium sulfate may be incorporated into the mixtures used to make the molding compounds used in the invention and will therefore be dispensed throughout the prosthesis. For example 0.7 micron barium sulfate particles comprising one to five percent by weight of the PMMA beads can be blended with the PMMA beads used in the process of the invention. These particles will coat the PMMA beads, producing an implant which has a sufficient X-ray density to produce a clear image on an X-ray film.

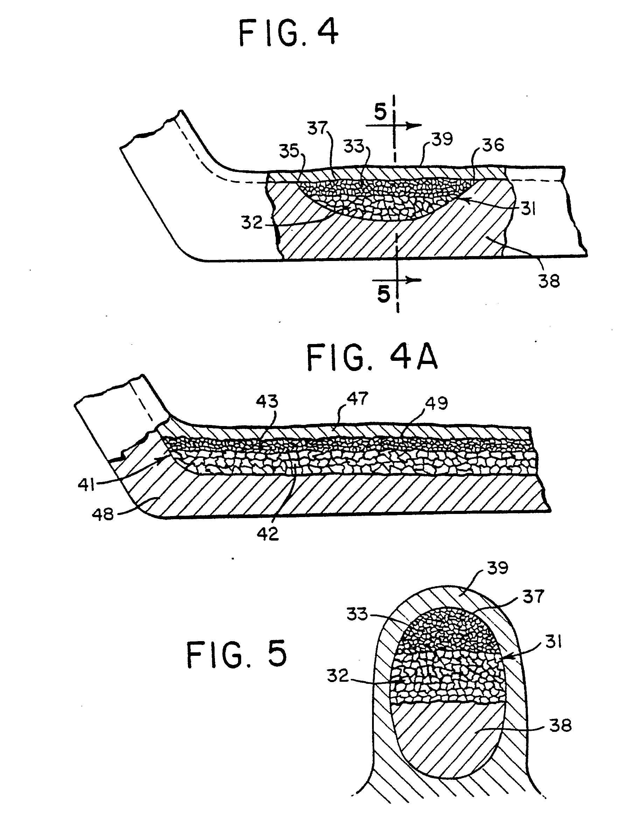

An implant according to the present invention may also be used to replace a missing section of jaw bone where deterioration has taken place. As shown in Figs. 4 and 4A. Referring to Fig. 4 an implant 31 having a region of coarse porosity 32 which interfaces with bone 38 and a region of fine porosity 33 which interfaces with gum tissue 39 may be produced by surgically opening the gum, taking an impression of jaw bone 38, and producing a mold. The impression may be shaped so that the implant smoothly fits with the existing jaw bone at its ends 35 and 36 and follows the contour or surface 37 that a jaw without signs of deterioration would have.

In addition implants produced according to the present invention can be used to build up edentulous ridges in patients with dentures. The building up of the vertical height of these ridges can augment the natural ridges of the jaw bone which exist when the teeth have been removed, preventing denture movement such as rocking and sliding. Such ridges may be incorporated on surface 37 of implant 31 of Fig. 4, or similarly on the implant of Fig. 4A. Alternatively such ridges may be formed by separate small implants on a jaw which has suffered less deterioration.

### Example 4
Procedure for producing jaw bone implantable additions for improved denture fitting.

In cases where the bone has receded due to long prior tooth extractions, the gum is surgically opened along the line of the jaw bone and an impression taken of the shape of the bone using Citricon silicone mold material. This mold is used to cast or manufacture a so-called Stone model (a hard plastic for dental use, well known in the art) by pouring Stone into the Citricon mold. Then the height of the resultant model is increased by adding hard wax or fast setting plastic to the cast until the model has increased in thickness and height by approximately one third to one half of the thickness of the pre-existing jaw bone. The top surface of the resultant model is rounded so that a cross section of the model has a convex upper surface and a concave lower surface that conforms to the shape of the jaw bone (See Fig. 5). The ends of the resultant model are tapered as shown in Fig. 4.

The model is then used to make a Citricon split mold, having the parting line midway between the top and bottom surfaces.

The bottom part of the mold is filled with HEMA-wetted coarse PMMA particles and heated dielectrically for a sufficient time (about two minutes) to weld the particles together. The top half of the mold is filled with HEMA-wetted PMMA fine particles, and the bottom filled mold is placed over the top mold so that the fine particles are pressed against the coarse particle molding. The combined, closed mold is then heated dielectrically for four to six minutes.

The sintered molding is then cooled and removed from the mold and placed in boiling water for two minutes. The molding thus treated is then placed over the deteriorated jaw bone so that it fits perfectly, and the gum is closed over the implant. Referring to Figs. 4 and 5 the coarse particle side 32, being in contact with the jaw bone 38, promotes bone attachment, while the fine particle side 33, being in contact with gum tissue 39, promotes gum attachment. After about 6 weeks of healing and attachment, dentures may be fitted.

Referring to Fig. 4A, an implant 41 for a jawbone 48 which has undergone much more extensive deterioration in a direction along the jawbone is shown. A region of coarse porosity 42 interfaces with jawbone 48, and a region of fine porosity 43 interfaces with gum tissue 47. Implant 41 can be produced following the procedure outlined above in Example 4. It is necessary to expose a longer segment of jawbone than would be necessary to produce the implant of Fig. 4. Ridges can be formed along surface 49 to enhance denture fit.

In those cases where some teeth or tooth roots remain to be extracted prior to fitting for a denture, it is advantageous to use the procedure as described in Example 1. The implanted porous roots are completely covered by the gum because no post is needed and after about 6 weeks, dentures can be fitted. This use of the freshly formed root sockets prevents bone recession and results in a better fitting denture, that will not slide or rock.

Modifications of Example 4 can be used to produce implants which serve as edentulous ridges when bone and gum tissue ingrowth result in attachment to an existing jawbone.

### Claims

1. A molded nonbiodegradable porous implantable oral prosthesis having an area of relatively coarse porosity (2, 12, 22, 32, 42) where the prosthesis is intended to interface with bone tissue (6, 16, 26, 38, 48) and relatively fine porosity where the prosthesis is intended to interface with soft tissue (7, 17, 27, 39, 47) characterised in that the prosthesis comprises sintered polymeric particles coated with a hydrophilic material.

2. A prosthesis as claimed in claim 1, molded to form a tooth root (1, 11).

3. A prosthesis as claimed in claim 1 or 2 further comprising a molded polymeric article shaped to serve as the crown (4, 14) of a tooth attached to said oral prosthesis.

4. A prosthesis as claimed in claim 3, in which said molded polymeric article which serves as a crown (4, 14) is comprised of an acrylic polymer.

5. A prosthesis as claimed in claim 1 molded to form an edentulous ridge.

6. A prosthesis as claimed in claim 1, molded to form a section (31, 41) of jaw bone.

7. A prosthesis as claimed in claim 6, comprising edentulous ridges.

8. A prosthesis as claimed in any preceding claim, in which said polymeric particles are comprised of an acrylic polymer.

9. A prosthesis as claimed in claim 8 in which said acrylic polymer is polymethylmethacrylate.

10. A prosthesis as claimed in any preceding claim, in which the hydrophilic material is a polymerized monomer.

11. A prosthesis as claimed in claim 10, in which said hydrophilic material is polyhydroxyethyl methacrylate.

12. A prosthesis as claimed in any preceding claim, in which the hydrophilic material is a polymerized solution of a monomer and a cross linking agent, said crosslinking agent comprising one tenth percent to five percent by weight of said solution.

13. A prosthesis as claimed in any of claims 1 to 10, in which said hydrophilic material comprises a polymerized solution of hydroxyethyl methacrylate and one tenth percent to five percent by weight of a methacrylic diester of ethylene glycol.

14. A prosthesis as claimed in claim 13 in which said methacrylic diester of ethylene glycol is selected from
a) tetraethyleneglycol dimethacrylate,
b) triethyleneglycol dimethacrylate,
c) diethyleneglycol dimethacrylate,
d) monoethyleneglycol dimethacrylate, and
e) mixtures thereof.

15. A prosthesis as claimed in any preceding claim, in which the area of relatively fine porosity is comprised of pores in the range of about 125 to 210 microns in diameter and the area of relatively coarse porosity is comprised of pores in the range of about 350 to 415 microns in diameter.

16. A prosthesis as claimed in any preceding claim further comprising a reinforcing core member (5, 15).

17. A prosthesis as claimed in claim 16 in which the reinforcing core member is a polar plastic.

18. A prosthesis as claimed in claim 17 in which the polar plastic is polymethylmethacrylate.

19. A prosthesis as claimed in any preceding claim, further comprising a radio-opaque material dispersed throughout the prosthesis.

20. A prosthesis as claimed in any of claims 1 to 5, further comprising a temporary post (28) of a nonpolar plastic with an end imbedded in said prosthesis.

21. A prosthesis as claimed in claim 20 in which the embedded end of said post is threaded.

22. A prosthesis as claimed in claim 20 or 21, in which the post is comprised of polyethylene.

23. A prosthesis as claimed in claim 20 or 21, in which the post is comprised of polypropylene.

24. A process for producing a porous implantable oral prosthesis comprising the steps of:
a) filling a portion of a mold cavity of the desired shape with relatively coarse polymeric particles (2, 12, 22, 32, 42) coated with a hydrophilic monomer where said prosthesis is intended to interface with bone tissue (6, 16, 26, 38, 48);
b) filling the remainder of said mold cavity with relatively fine polymeric particles (3, 13, 23, 33, 43) coated with a hydrophilic monomer where said prosthesis is intended to interface with soft tissue (7, 17, 27, 39, 47);
c) sintering the polymeric particles and polymerizing the hydrophilic monomer in the mold by the application of heat to form the prosthesis;

d) allowing the mold to cool;
e) removing the sintered prosthesis from the mold; and
f) placing the prosthesis in a hot liquid to remove toxic residues.

25. A process as claimed in claim 24, in which said oral prosthesis is a tooth root and said process includes the preliminary steps of:
(a) cleaning an extracted tooth; and
(b) producing a mold with a cavity shaped to conform to the extracted tooth root.

26. A process as claimed in claim 25 comprising the additional step of placing the cleaned extracted tooth in molten wax to form a thin conformal coating on said tooth before producing said mold.

27. A process as claimed in claim 24 in which said oral prosthesis is a tooth root for use where bone and gum have receded including the preliminary steps of:
(a) taking an impression of a conically shaped hole formed in an exposed jaw bone;
(b) making an impression of said hole using a fast setting, low shrinkage material; and
(c) making a mold with a cavity in the shape of said impression.

28. A process as claimed in claim 27 comprising the additional step of attaching a crown (4, 14) to the porous oral prosthesis to form an entire tooth.

29. A process as claimed in any of claims 24 to 28, comprising the additional step of inserting an end of a temporary post (28) comprised of a nonpolar plastic into said mold cavity before sintering or immediately thereafter.

30. A process as claimed in any of claims 24 to 28, comprising the additional step of inserting a reinforcing core member (5, 15) in said mold cavity before sintering or immediately therafter.

31. A process as claimed in claim 24, in which said prosthesis is for use as a portion of jaw bone, said process comprising the preliminary steps of:
(a) taking an impression of an exposed jaw bone;
(b) using said impression to cast a model of the jaw bone;
(c) adding additional material to said model to form a desired shape; and
(d) producing a mold with a cavity of said desired shape.

32. A process as claimed in claim 31 in which the desired shape is formed to include ridges to facilitate a denture fit.

33. A process for producing an implantable tooth having porous root and a solid crown comprising the steps of:
(a) cleaning an extracted tooth;
(b) producing a first mold with a cavity shaped to conform to the extracted tooth root;
(c) forming a second mold with a cavity in the shape of the crown portion of the extracted tooth protruding from said first mold;
(d) removing the extracted tooth from the molds;
(e) filling a portion of said first mold cavity with relatively coarse polymeric particles (2, 12) coated with a hydrophilic monomer where the root is intended to interface with bone tissue (6, 16);

(f) filling the remainder of said first mold with relatively fine polymeric particles (3, 13) coated with a hydrophilic monomer where the root is intended to interface with soft tissue (7, 17);

(g) sintering the polymeric particles and polymerizing the hydrophilic monomer in the first mold by the application of heat;

(h) allowing the first mold to cool;

(i) filling the cavity of the second mold with a polymerizable liquid;

(j) placing the first mold over the second mold in proper alignment for the cavities to form a shape identical to the extracted tooth, and to allow a portion of said polymerizable liquid to penetrate said root;

(k) polymerizing the liquid;

(l) removing the tooth from the mold; and

(m) placing the tooth in a hot liquid to remove toxic residue.

34. A process as claimed in claim 33 comprising the additional step of applying fluid pressure during the polymerization of said liquid.

35. A process as claimed in claim 33 or 34, comprising the additional step of inserting a reinforcing core member in said first mold cavity before sintering or immediately thereafter.

36. A process as claimed in any of claims 24 to 35, in which the step of sintering the polymeric particles and polymerizing the hydrophilic monomer is carried on at atmospheric pressure.

37. A process as claimed in any of claims 24 to 36 in which the polymeric particles are comprised of polymethyl methacrylate.

38. A process as claimed in any of claims 24 to 37, in which the sintering is performed by heating in a dielectric oven.

39. A process as claimed in any of claims 24 to 38, in which the sintering is performed at a temperature in the range of from about 175°C to about 225°C.

40. A process as claimed in any of claims 24 to 39, in which the hot liquid is boiling water.

41. A process as claimed in any of claims 24 to 40 in which the hydrophilic monomer is hydroxyethyl methacrylate.

42. A process as claimed in any of claims 24 to 41, in which the hydrophilic monomer is combined with a cross-linking agent comprising one tenth percent to five percent by weight of the monomer.

43. A process as claimed in any of claims 24 to 42, in which the hydrophilic monomer is hydroxyethyl methacrylate combined with a crosslinking agent comprising one tenth percent to five percent by weight of the monomer.

44. A process as claimed in claim 43, in which the crosslinking agent is a methacrylic diester of ethylene glycol.

45. A process as claimed in claim 44, in which the methacrylic diester of ethylene glycol is selected from

(a) tetraethyleneglycol dimethacrylate,

(b) triethyleneglycol dimethacrylate,

(c) diethyleneglycol dimethacrylate,

(d) monoethyelenglycol dimethacrylate, and

(e) mixtures thereof.

**Patentansprüche**

1. Geformte, biologisch nicht abbaubare, poröse, implantierbare Mundprothese mit einem Gebiet relativ grober Porosität (2, 12, 22, 32, 42), wo die Prothese an Knochengewebe (6, 16, 26, 38, 48) anliegen soll, und relativ feiner Porosität, wo sie an weichem Gewebe (7, 17, 27, 39, 47) anliegen soll, dadurch gekennzeichnet, dass die Prothese aus gesinterten, mit einem hydrophilen Material überzogenen polymeren Teilchen besteht.

2. Prothese nach Anspruch 1, geformt in Gestalt einer Zahnwurzel (1, 11).

3. Prothese nach Anspruch 1 oder 2, ferner enthaltend einen geformten polymeren Körper, der zur Verwendung als Krone (4, 14) eines an dieser Mundprothese befestigten Zahns gestaltet ist.

4. Prothese nach Anspruch 3, worin dieser geformte, als Krone (4, 14) dienende polymere Artikel aus einem Acrylpolymeren besteht.

5. Prothese nach Anspruch 1, geformt, zur Bildung eines zahnlosen Wulsts.

6. Prothese nach Anspruch 1, geformt zur Bildung eines Kieferknochenabschnitts (31, 41).

7. Prothese nach Anspruch 6, bestehend aus zahnlosen Wulsten.

8. Prothese nach einem der vorhergehenden Ansprüche, worin diese polymeren Teilchen aus einem Acrylpolymeren bestehen.

9. Prothese nach Anspruch 8, worin als solches Acrylpolymer Polymethylmethacrylat vorliegt.

10. Prothese nach einem der vorhergehenden Ansprüche, worin das hydrophile Material ein polymerisiertes Monomer ist.

11. Prothese nach Anspruch 10, worin als solches hydrophiles Material Polyhydroxyäthylmethacrylat vorliegt.

12. Prothese nach einem der vorhergehenden Ansprüche, worin das hydrophile Material eine polymerisierte Lösung eines Monomeren und eines Vernetzungsmittels ist, wobei dieses Vernetzungsmittel 0,1 bis 5 Gewichtsprozent dieser Lösung ausmacht.

13. Prothese nach einem der Ansprüche 1 bis 10, worin dieses hydrophile Material aus einer polymerisierten Lösung von Hydroxyäthylmethacrylat und 0,1 bis 5 Gewichtsprozent eines Äthylenglykol-Methacrylsäurediesters besteht.

14. Prothese nach Anspruch 13, worin dieser Äthylenglykol-methacrylsäurediester unter

a) Tetraäthylenglykol-dimethacrylat,

b) Triäthylenglykol-dimethacrylat,

c) Diäthylenglykol-dimethylacrylat,

d) Monoäthylenglykol-dimethacrylat und

e) deren Gemischen

ausgewählt ist.

15. Prothese nach einem der vorhergehenden Ansprüche, worin das Gebiet relativ feiner Porosität aus Poren im Bereich von etwa 125 bis 210 Mikron Durchmesser und das Gebiet relativ grober Porosität aus Poren im Bereich von etwa 350 bis 415 Mikron Durchmesser besteht.

16. Prothese nach einem der vorhergehenden Ansprüche, ferner enthaltend ein verstärkendes Kernstück (5, 15).

17. Prothese nach Anspruch 16, worin als verstärkendes Kernstück ein polarer Kunststoff vorliegt.

18. Prothese nach Anspruch 17, worin als polarer Kunststoff Polymethylmethacrylat vorliegt.

19. Prothese nach einem der vorhergehenden Ansprüche, ferner enthaltend ein über die ganze Prothese verteiltes röntgenundurchlässiges Material.

20. Prothese nach einem der Ansprüche 1 bis 5, ferner enthaltend einen provisorischen Stift (28) aus unpolarem Kunststoff mit einem in dieser Prothese eingebetteten Ende.

21. Prothese nach Anspruch 20, worin das eingebettete Ende dieses Stifts mit einem Gewinde versehen ist.

22. Prothese nach Anspruch 20 oder 21, worin der Stift aus Polyäthylen besteht.

23. Prothese nach Anspruch 20 oder 21, worin der Stift aus Polypropylen besteht.

24. Verfahren zur Herstellung einer porösen implantierbaren Mundprothese, bei dem man nacheinander

a) einen Teil eines Formhohlraums gewünschter Gestalt mit relativ großen, mit einem hydrophilen Monomeren überzogenen polymeren Teilchen (2, 12, 22, 32, 42) füllt, wo diese Prothese an Knochengewebe anliegen soll (6, 16, 26, 38, 48),

b) den Rest dieses Formhohlraums mit relativ feinen, mit einem hydrophilen Monomeren überzogenen polymeren Teilchen (3, 13, 23, 33, 43) füllt, wo diese Prothese an weichem Gewebe (7, 17, 27, 39, 47) anliegen soll,

c) die polymeren Teilchen sintert und das hydrophile Monomere in der Form durch Wärmezufuhr zur Bildung der Prothese polymerisiert,

d) die Form abkühlen lässt,

e) die gesinterte Prothese aus der Form entnimmt und

f) die Prothese zur Entfernung toxischer Rückstände in eine heisse Flüssigkeit einlegt.

25. Verfahren nach Anspruch 24, bei dem diese Mundprothese eine Zahnwurzel ist, einschliesslich der vorbereitenden Stufen, das man

a) einen gezogenen Zahn reinigt und

b) eine Form mit einem Hohlraum herstellt, dessen Gestalt mit der gezogenen Zahnwurzel übereinstimmt.

26. Verfahren nach Anspruch 25, bei dem man zusätzlich den gereinigten gezogenen Zahn in geschmolzenes Wachs einlegt, um auf diesem Zahn vor der Herstellung jener Form einen dünnen Überzug derselben Gestalt zu bilden.

27. Verfahren nach Anspruch 24, bei dem diese Mundprothese eine Zahnwurzel zur Verwendung bei Knochen- und Zahnfleischschwund ist, einschliesslich der vorbereitenden Stufen, dass man:

a) einen Abdruck eines kegelförmigen, in freiliegendem Kieferknochen gebildeten Lochs nimmt,

b) einen Abdruck dieses Lochs mit einem schnell abbindenden Material geringer Schwindung nimmt und

c) eine Form mit einem Hohlraum in der Gestalt dieses Abdrucks herstellt.

28. Verfahren nach Anspruch 27, bei dem man zusätzlich eine Krone (4, 14) an die poröse Mundprothese zur Bildung eines kompletten Zahns abringt.

29. Verfahren nach einem der Ansprüche 24 bis 28, wobei man zusätzlich ein Ende eines aus unpolarem Kunststoff bestehenden provisorischen Stifts (28) vor oder unmittelbar nach dem Sintern in jenen Formhohlraum einbringt.

30. Verfahren nach einem der Ansprüche 24 bis 28, bei dem man zusätzlich ein verstärkendes Kernstück (5, 15) vor oder unmittelbar nach dem Sintern in jeden Formhohlraum einbringt.

31. Verfahren nach Anspruch 24, bei dem diese Prothese als Teil eines Keiferknochens dienen, soll, einschliesslich der vorbereitenden Stufen, dass man

a) einen Abdruck eines freiliegenden Kieferknochens nimmt,

b) diesen Abdruck zum Giessen eines Modells des Kieferknochens verwendet,

c) auf dieses Modell zur Bildung der gewünschten Gestalt weiteres Material aufbringt und

d) eine Form mit einem Hohlraum dieser gewünschten Gestalt herstellt.

32. Verfahren nach Anspruch 31, bei dem man die gewünschte Gestalt mit Wülsten zur besseren Passung einer Dentalprothese versieht.

33. Verfahren zur Herstellung eines implantierbaren Zahns mit einer porösen Wurzel und massiven Krone, bei dem man nacheinander:

a) einen gezogenen Zahn reinigt,

b) eine erste Form mit einem Hohlraum herstellt, dessen Gestalt mit der gezogenen Zahnwurzel übereinstimmt,

c) eine zweite Form mit einem Hohlraum in Gestalt des Kronenteils des gezogenen, aus jener ersten Form vorstehenden Zahns bildet,

d) den gezogenen Zahn aus den Formen herausnimmt,

e) einen Teil dieses ersten Formhohlraums mit relativ groben, mit einem hydrophilen Monomeren überzogenen polymeren Teilchen (2, 12) füllt, wo die Wurzel an Knochengewebe (6, 16) anliegen soll,

f) den Rest dieser ersten Form mit relativ feinen, mit einem hydrophilen Monomer überzogenen polymeren Teilchen (3, 13) füllt, wo die Wurzel an weichem Gewebe (7, 17) anliegen soll,

g) die polymeren Teilchen sintert und das hydrophile Monomere in der ersten Form durch Wärmezufuhr polymerisiert,

h) die erste Form abkühlen lässt,

i) den Hohlraum der zweiten Form mit einer polymerisierbaren Flüssigkeit füllt,

j) die erste Form so ausgerichtet über die zweite Form setzt, dass die Hohlräume eine mit dem gezogenen Zahn identische Gestalt bilden, und einen Teil dieser polymerisierbaren Flüssigkeit jene Wurzel durchdringen lässt,

k) die Flüssigkeit polymerisert,

l) den Zahn aus der Form entnimmt und

m) den Zahn zur Entfernung toxischer Rückstände in eine heisse Flüssigkeit einlegt.

34. Verfahren nach Anspruch 33, bei dem man zusätzlich während der Polymerisation jener Flüssigkeit hydraulischen Druck anlegt.

35. Verfahren nach Anspruch 33 oder 34, bei dem man zusätzlich ein verstärkendes Kernstück vor oder unmittelbar nach dem Sintern in jenen ersten Formhohlraum einbringt.

36. Verfahren nach einem der Ansprüche 24 bis 35, bei dem der Vorgang des Sinterns der polymeren Teilchen und der Polymerisation des hydrophilen Monomeren bei Atmosphärendruck erfolgt.

37. Verfahren nach einem der Ansprüche 24 bis 36, bei dem die polymeren Teilchen aus Polymethylmethacrylat bestehen.

38. Verfahren nach einem der Ansprüche 24 bis 34, bei dem das Sintern durch Erhitzen in einem Dielektrischen Ofen erfolgt.

39. Verfahren nach einem der Ansprüche 24 bis 38, bei dem das Sintern bei einer Temperatur im Bereich von etwa 175°C bis etwa 225°C erfolgt.

40. Verfahren nach einem der Ansprüche 24 bis 39, bei dem die heisse Flüssigkeit kochendes Wasser ist.

41. Verfahren nach einem der Ansprüche 24 bis 40, bei dem als hydrophiles Monomeres Hydroxyäthylmethacrylat vorliegt.

42. Verfahren nach einem der Ansprüche 24 bis 41, bei dem das hydrophile Monomere mit einem Vernetzungsmittel kombiniert ist, das 0,1 bis 5 Gewichtsprozent des Monomeren ausmacht.

43. Verfahren nach einem der Ansprüche 24 bis 42, bei dem das hydrophile Monomere Hydroxyäthylmethacrylat kombiniert mit einem Vernetzungsmittel ist, das 0,1 bis 5 Gewichtsprozent des Monomeren ausmacht.

44. Verfahren nach Anspruch 43, bei dem als Vernetzungsmittel ein Äthylenglykol-methacrylsäurediester vorliegt.

45. Verfahren nach Anspruch 44, bei dem der Äthylenglykol-methacrylsäurediester unter
a) Tetraäthylenglykol-dimethacrylat,
b) Triäthylenglykol-dimethacrylat,
c) Diäthylenglykol-dimethylacrylat,
d) Monoäthylenglykol-dimethacrylat und
e) deren Gemischen
ausgewählt ist.

## Revendications

1. Une prothèse orale poreuse implantable, moulée et non biodégradable, ayant une surface de porosité relativement grossière (2, 12, 22, 32, 42) là où la prothèse est destinée à se trouver en contact avec du tissu osseux (6, 16, 26, 38, 48), et de porosité relativement fine là où la prothèse est destinée à se trouver en contact avec du tissu mou (7, 17, 27, 39, 47) caractérisée en ce que la prothèse comprend des particules polymères frittées revêtues d'un matériau hydrophile.

2. Une prothèse selon la revendication 1, moulée afin de former une racine de dent (1, 11).

3. Une prothèse selon la revendication 1 ou 2, comprenant en outre un objet polymère moulé façonnée pour servir de couronne (4, 14) à une dent fixée à ladite prothèse orale.

4. Une prothèse selon la revendication 3, dans laquelle ledit objet polymère moulé servant de couronne (4, 14) est constitué d'un polymère acrylique.

5. Une prothèse selon la revendication 1, moulée afin de former une crête édentée.

6. Une prothèse selon la revendication 1, moulée afin de former une partie (31, 41) d'os maxillaire.

7. Une prothèse selon la revendication 6, comprenant des crêtes édentées.

8. Une prothèse selon l'une quelconque des revendications précédentes, dans laquelle lesdites particules polymères sont constituées d'un polymère acrylique.

9. Une prothèse selon la revendication 8, dans laquelle ledit polymère acrylique est le polyméthacrylate de méthyle.

10. Une prothèse selon l'une quelconque des revendications précédentes, dans laquelle le matériau hydrophile est un monomère polymérisé.

11. Une prothèse selon la revendication 10, dans laquelle ledit matériau hydrophile est le polyméthacrylate d'hydroxyéthyle.

12. Une prothèse selon l'une quelconque des revendications précédentes, dans laquelle le matériau hydrophile est une solution polymérisée d'un monomère et d'un agent de réticulation, ledit agent de réticulation représentant un dixième de pour cent à cinq pour cent en poids de ladite solution.

13. Une prothèse selon l'une quelconque des revendications 1 à 10, dans laquelle ledit matériau hydrophile comprend une solution polymérisée de méthacrylate d'hydroxyéthyle et un dixième de pour cent à cinq pour cent en poids d'un diester méthacrylique d'éthylène glycol.

14. Une prothèse selon la revendication 13, dans laquelle ledit diester méthacrylique d'éthylène glycol est sélectionné parmi
a) le diméthacrylate de tétraéthylène glycol,
b) le diméthacrylate de triéthylène glycol,  ·
c) le diméthacrylate de diéthylène glycol,
d) le diméthacrylate de monoéthylène glycol, et
e) leurs mélanges.

15. Une prothèse selon l'une quelconque des revendications précédentes, dans laquelle la surface de porosité relativement fine est constituée de pores dont le diamètre est compris dans l'intervalle d'environ 125 à 210 microns et la surface de porosité relativement grossière est constituée de pores dont le diamètre est compris dans l'intervalle d'environ 350 à 415 microns.

16. Une prothèse selon l'une quelconque des revendications précédentes, comprenant en outre un élément de coeur de renforcement (5, 15).

17. Une prothèse selon la revendication 16, dans laquelle l'élément de coeur de renforcement est une matière plastique polaire.

18. Une prothèse selon la revendication 17, dans laquelle la matière plastique polaire est le polyméthacrylate de méthyle.

19. Une prothèse selon l'une quelconque des revendications précédentes, comprenant en outre un matériau opaque aux rayonnements dispersé dans la prothèse toute entière.

20. Une prothèse selon l'une quelconque des revendications 1 à 5, comprenant en outre un pivot temporaire (28) en matière plastique non polaire dont une extrémité est implantée dans ladite prothèse.

21. Une prothèse selon la revendication 20, dans laquelle l'extrémité implantée dudit pivot est filetée.

22. Une prothèse selon la revendication 20 ou 21, dans laquelle ledit pivot est constitué de polyéthylène.

23. Une prothése selon la revendication 20 ou 21, dans laquelle ledit pivot est constitué de polypropylène.

24. Un procédé de production d'une prothèse orale poreuse implantable, comprenant les étapes suivantes:

a) remplir une partie de la matrice d'un moule de forme requise de particules polymères relativement grossières (2, 12, 22, 32, 42) revêtues d'un monomère hydrophile là où ladite prothèse est destinée à se trouver en contact avec du tissu osseux (6, 16, 26, 38, 48);

b) remplir le reste de ladite matrice du moule de particules polymères relativement fines (3, 13, 23, 33, 43) revêtues d'un monomère hydrophile là où ladite prothèse est destinée à se trouver en contact avec du tissu mou (7, 17, 27, 39, 47);

c) fritter les particules polymères et polymériser le monomère hydrophile dans le moule par application de chaleur afin de former la prothèse;

d) laisser le moule refroidir;

e) sortir la prothèse frittée du moule; et

f) placer la prothèse dans un liquide chaud afin d'éliminer les résidus toxiques.

25. Un procédé selon la revendication 24, dans lequel ladite prothèse orale est une racine de dent et ledit procédé comprend les étapes préalables suivantes:

a) nettoyer une dent arrachée; et

b) produire un moule ayant une matrice formée pour se conformer à la racine de dent arrachée.

26. Un procédé selon la revendication 25 oomprenant l'étape supplémentaire de placer la dent arrachée nettoyée dans de la cire fondue pour former un enduit conforme de faible épaisseur sur ladite dent avant de produire ledit moule.

27. Un procédé selon la revendication 24, dans lequel ladite prothèse orale est une racine de dent à utiliser là où l'os et la gencive ont reculé comprenant les étapes préalables suivantes:

a) prendre une empreinte d'une cavité de forme conique formée dans un os maxillaire exposé;

b) produire une empreinte de ladite cavité au moyen d'un matériau à prise rapide et de faible retrait; et

c) produire un moule ayant une matrice en forme de ladite empreinte.

28. Un procédé selon la revendication 27, comprenant l'étape supplémentaire d'attacher une couronne (4, 14) à la prothèse orale poreuse afin de former une dent entière.

29. Un procédé selon l'une quelconque des revendications 24 à 28, comprenant l'étape supplémentaire d'introduire une extrémité d'un pivot temporaire (28) constituée d'une matière plastique non polaire dans ladite matrice du moule avant le frittage ou immédiatement après celui-ci.

30. Un procédé selon l'une quelconque des revendications 24 à 28, comprenant l'étape supplémentaire d'introduire un élément de coeur de renforcement (5, 15) dans ladite matrice du moule avant le frittage ou immédiatement après celui-ci.

31. Un procédé selon la revendication 24, dans lequel ladite prothèse est destinée à être utilisée comme une partie d'os maxillaire, ledit procédé comprenant les étapes préalables suivantes:

a) prendre une empreinte d'un os maxillaire exposé;

b) utiliser ladite empreinte afin de couler un modèle de l'os maxillaire;

c) ajouter du matériau supplémentaire audit modèle pour obtenir une forme requise; et

d) produire un moule ayant une matrice de ladite forme requise.

32. Un procédé selon la revendication 31, dans lequel la forme requise est exécutée de manière à incorporer des crêtes pour faciliter l'adaptation d'un dentier.

33. Un procédé de production d'une dent implantable ayant une racine poreuse et une couronne solide comprenant les étapes suivantes:

a) nettoyer une dent arrachée;

b) produire un premier moule ayant une matrice formée pour se conformer à la racine de la dent arrachée;

c) former un deuxième moule ayant une matrice en forme de la partie couronne de la dent arrachée faisant saillie dudit premier moule;

d) sortir la dent arrachée des moules;

e) remplir une partie de ladite matrice du premier moule de particules polymères relativement grossières (2, 12) revêtues d'un monomère hydrophile là où la racine est destinée à se trouver en contact avec du tissu osseux (6, 16);

f) remplir le reste dudit premier moule de particules polymères relativement fines (3, 13) revêtues d'un monomère hydrophile là où la racine est destinée à se trouver en contact avec du tissu mou (7, 17);

g) fritter les particules polymères et polymériser le monomère hydrophile dans le premier moule par application de chaleur;

h) laisser le premier moule refroidir;

i) remplir la matrice du deuxième moule d'un liquide polymérisable;

j) placer le premier moule sur le deuxième moule dans l'alignement convenable pour que les matrices produisent une forme identique à la dent arrachée et permettent à une partie dudit liquide polymérisable de pénétrer dans ladite racine;

k) polymériser le liquide;

l) sortir la dent du moule; et

m) placer la dent dans un liquide chaud afin d'éliminer les résidus toxiques.

34. Un procédé selon la revendication 33, comprenant l'étape supplémentaire d'appliquer une pression de fluide pendant la polymérisation dudit liquide.

35. Un procédé selon la revendication 33 ou 34, comprenant l'étape supplémentaire d'introduire un élément de coeur de renforcement dans ladite matrice du premier moule avant le frittage ou immédiatement après celui-ci.

36. Un procédé selon l'une quelconque des revendications 24 à 35, dans lequel l'étape de frittage des particules polymères et de polymérisation du monomère hydrophile est exécutée à la pression atmosphérique.

37. Un procédé selon l'une quelconque des revendications 24 à 36, dans lequel les particules polymères sont constituées de polyméthacrylate de méthyle.

38. Un procédé selon l'une quelconque des revendications 24 à 37, dans lequel le frittage est affectué par chauffage dans un four diélectrique.

39. Un procédé selon l'une quelconque des revendications 24 à 38, dans lequel le frittage est effectué à une température comprise dans l'intervalle d'environ 175°C à environ 225°C.

40. Un procédé selon l'une quelconque des revendications 24 à 39, dans lequel le liquide chaud est l'eau bouillante.

41. Un procédé selon l'une quelconque des revendications 24 à 40, dans lequel le monomère hydrophile est le méthacrylate d'hydroxyéthyle.

42. Un procédé selon l'une quelconque des revendications 24 à 41, dans lequel le monomère hydrophile est en combinaison avec un agent de réticulation représentant un dixième de pour cent à cinq pour cent en poids du monomère.

43. Un procédé selon l'une quelconque des revendications 24 à 42, dans lequel le monomère hydrophile est le méthacrylate d'hydroxyéthyle en combinaison avec un agent de réticulation représentant un dixième de pour cent à cinq pour cent en poids du monomère.

44. Un procédé selon la revendication 43, dans lequel l'agent de réticulation est un diester méthacrylique d'éthylène glycol.

45. Un procédé selon la revendication 44, dans lequel le diester méthacrylique d'éthylène glycol est sélectionné parmi
   a) le diméthacrylate de tétraéthylène glycol,
   b) le diméthacrylate de triéthylène glycol,
   c) le diméthacrylate de diéthylène glycol,
   d) le diméthacrylate de monoéthylène glycol, et
   e) leurs mélanges.

0 053 903

FIG. 1

FIG. 2

FIG. 3

1

# FIG. 4

# FIG. 4A

# FIG. 5